# EUROPEAN PATENT APPLICATION

(11) **EP 0 725 139 A2**
(43) Date of publication of application: **07.08.1996**
(21) Application number: 96101304.2
(22) Date of filing: 31.01.1996
(51) Int. Cl.: C12N 15/57, C12N 9/64, G01N 33/573

(54) **Recombinant prostate-specific antigen and its use as an immunoassay calibrator**

(30) Priority: 03.02.1995 US 383111; 28.04.1995 US 430498
(71) Applicant: Bayer Corporation, Pittsburgh, PA 15219-2502 (US)
(72) Inventor: Yeung, Kwok K., Ridgefield, Connecticut 06877 (US); Barnett, Thomas R., Prospect, Connecticut 06712 (US); Ng, Phillip C., Woodside, New York 11377 (US)
(74) Representative: Kröger, Bernd, Dr.

(57) **Abstract**

Recombinant PSA (rPSA), including immunologically cross-reactive fragments, and its use as an immunoassay calibrator. rPSA is free-form PSA, uncomplexed with proteolytic inhibitors, and shows immunoreactivity in commercial PSA tests.

SEQUENCE LISTING

(1) GENERAL INFORMATION:

(i) APPLICANT: Kwok K. Yeung, Thomas R. Barnett and Phillip C. Ng

(ii) TITLE OF INVENTION: Recombinant Prostate-Specific Antigen
and Its Use as an Immunoassay Calibrator

(iii) NUMBER OF SEQUENCES: 3

(iv) CORRESPONDENCE ADDRESS:

(A) ADDRESSEE: Bayer Corporation
(B) STREET: 511 Benedict Avenue
(C) CITY: Tarrytown
(D) STATE: New York
(E) COUNTRY: USA
(F) ZIP: 10591

(v) COMPUTER READABLE FORM:

(A) MEDIUM TYPE: Diskette, 3.50 inch, 1.44 Mb storage
(B) COMPUTER TYPE: IBM PS/2 compatible
(C) OPERATING SYSTEM: MS-DOS
(D) SOFTWARE: WordPerfect 5.1

(vi) CURRENT APPLICATION DATA:

(A) APPLICATION NUMBER:
(B) FILING DATE:
(C) CLASSIFICATION:

(vii) PRIOR APPLICATION DATA:

(A) APPLICATION NUMBER: 08/383,111
(B) FILING DATE: 03-Feb-1995

(viii) ATTORNEY/AGENT INFORMATION:

(A) NAME: Andrew L. Klawitter
(B) REGISTRATION NUMBER: 26,557
(C) REFERENCE/DOCKET NUMBER: MST-1940.1

(ix) TELECOMMUNICATION INFORMATION:

(A) TELEPHONE: (914) 524-2093
(B) TELEFAX: (914) 524-3594

(2) INFORMATION FOR SEQUENCE ID NO:1

(i) SEQUENCE CHARACTERISTICS:

(A) LENGTH: 45 nucletides
(B) TYPE: nucleic acid
(C) STRANDEDNESS: single
(D) TOPOLOGY: linear

(ii) MOLECULE TYPE: synthetic polynucleotide

(iii) HYPOTHETICAL: no

(xi) SEQUENCE DESCRIPTION: SEQ ID NO:1

GGATCCTGTG TCACCATGTG GGTCCCGGTT GTCTTCCTCA CCCTG

(3) INFORMATION FOR SEQUENCE ID NO:2

(i) SEQUENCE CHARACTERISTICS:

(A) LENGTH: 94 nucletides
(B) TYPE: nucleic acid
(C) STRANDEDNESS: single
(D) TOPOLOGY: linear

(ii) MOLECULE TYPE: synthetic polynucleotide

(iii) HYPOTHETICAL: no

(xi) SEQUENCE DESCRIPTION: SEQ ID NO:2

GGATCCTGTG TCACCATGTG GGTCCCGGTT GTCTTCCTCA CCCTGTCCGT 50

GACGTGGATT GGTGCTATT GTGGGAGGCT GGGAGAAGCA TTCC

(4) INFORMATION FOR SEQUENCE ID NO:3

(i) SEQUENCE CHARACTERISTICS:

(A) LENGTH: 68 nucletides
(B) TYPE: nucleic acid
(C) STRANDEDNESS: single
(D) TOPOLOGY: linear

(ii) MOLECULE TYPE: synthetic polynucleotide

(iii) HYPOTHETICAL: no

(xi) SEQUENCE DESCRIPTION: SEQ ID NO:3

GCGGCCGCA TCATTAATGG TGATGGTGAT GATGGGGTT GGCCACGATG 50

GTGTCCTTG ATCCACTT

## Description

### CROSS-REFERENCE TO RELATED APPLICATION

This is a continuation-in-part of application Serial No. 08/383,111, filed February 3, 1995.

### BACKGROUND OF THE INVENTION

The present invention relates to polypeptides expressed by recombinant DNA means which are immunologically cross-reactive with human prostate-specific antigen (PSA). Further, the present invention concerns the use of recombinant PSA (rPSA) as a standard to calibrate PSA immunoassay methods.

Prostate-specific antigen (PSA) is a glycoprotein of about 33 kDa (Wang MC, Valenzuela LA, Murphy GP, and Chu TM. Purification of a human prostate specific antigen. *Invest Urol* 1979, 17:159-63) and has been shown to have amino acid homology to the kallikrein family of proteins (Watt KWK, Lee P-J, Timkulu TM, Chan WP, and Loor R. Human prostate-specific antigen: structure and functional similarity with serine proteases. *Proc Natl Acad Sci USA* 1986, 83:3166-70). PSA has proteolytic enzyme activity and has been found to be a serine protease. (Akiyama K, Nakamura T, Iwanaga S, and Hara M. The chymotrypsin-like activity of human prostate-specific antigen, r-seminoprotein. *FEBS Letters* 1987, 225:168-72; Christensson A, Laurell C-B, and Lilja H. Enzymatic activity of prostate-specific antigen and its reactions with extracellular serine proteinase inhibitors. *Eur J Biochem* 1990, 194:755-63). PSA is secreted by the epithelial cells of the prostate gland and is one of the major proteins found in seminal fluid (Lilja H, and Abrahamsson P-A. Three predominant proteins secreted by the human prostate gland. *Prostate* 1988,12:29-38). The function of PSA has been proposed for liquefaction of the seminal clot formed following ejaculation (Lilja H. A kallikrein-like serine protease in prostatic fluid cleaves the predominant seminal vesicle protein. *J Clin Invest* 1985, 76:1899-903).

In 1980, Papsidero *et al* first reported that PSA was found in sera of prostate cancer patients (Papsidero LD, Wang MC, Valenzuela LA, Murphy GP, and Chu TM. A prostate antigen in sera of prostate cancer patients. *Cancer Res* 1980 40:2428-32). Since then, numerous reports have been published on the use of this protein as a marker for diagnosis and management of prostate cancer (See reviews - Lage PH. Prostate specific antigen in diagnosis and management of prostate cancer. *Supplement to Urology* 1990 XXXVI:25-29; Oesterling JE. Prostate specific antigen: a critical assessment of the most useful tumor marker for adenocarcinoma of the prostate. *J Urol* 1991 145:907-23; and Armbruster DA. Prostate-specific antigen: biochemistry, analytical methods and clinical application. *Clin Chem* 1993 39:181-95). Recently, several investigators have reported on the value of PSA as a screening marker for prostate cancer (Catalona WJ, Smith DS, Ratliff TL, Dodd KM, Coplen DE, Yuan JJJ, Petros JA, and Andriole GL. Measurement of prostate-specific antigen in serum as a screening test for prostate cancer. *N Engl J Med* 1991 324:1156-61; Brawer MK, Chetner MP, Beatie J, Buchner DM, Vessella RL, and Lange PH. Screening for prostatic carcinoma with prostate specific antigen. *J Urol* 1992, 147:841-5). In fact, the U.S. Food and Drug Administration has recently approved a commercial kit for the early detection of prostate cancer when used in conjunction with digital rectal examination (M.D.D.I reports "The Gray Sheet" September 5, 1994, pp12-3).

Prostate-specific antigen has been demonstrated, *in vitro*, to form complexes with α₁-antichymotrypsin (ACT) in a molar stoichiometry of 1:1, (Christensson *et al*, *supra*). These complexes are stable to sodium dodecyl sulfate denaturation. When added to plasma, PSA formed complexes with both ACT and α₂-macroglobulin, although later reports showed that the predominant complex formed is with ACT (Stenman U-H, Leinonen J, Alfthan H, Rannikko S, Tuhkanen K, and Alfthan O. A complex between prostate-specific antigen and α₁-antichymotrypsin is the major form of prostate-specific antigen in serum of patients with prostatic cancer: assay of the complex improves clinical sensitivity for cancer. *Cancer Res* 1991 51:222-26; Lilja H, Christensson A, Dahlen U, Matikainen M-T, Nilsson O, Pettersson K, and Lovgren T. Prostate-specific antigen in sea occurs predominantly in complex with α₁-antichymotrypsin. *Clin Chem* 1991 37:1618-25).

A number of commercial kits for human blood PSA determination are presently being marketed. Some such kits have been shown to react differently to free or protease inhibitor-complexed PSA (Zhou AM, Tewari PC, Bluestein BL, Caldwell GW, and Larsen FL. Multiple forms of prostate-specific antigen in serum: differences in immunorecognition by monoclonal and polyclonal assays. *Clin Chem* 1993 39:2483-91). In fact, there are reports that one commercial kit detects PSA levels two to three times higher than other methods (Zhou *et al*, *supra*). One explanation for this difference is that the methods for making calibration standards differ among kit manufacturers. Some manufacturers use bovine serum albumin (BSA) as a matrix and others use human serum. Since serum contains protease inhibitors such as ACT and α₂-macroglobulin which complex with PSA, some epitopes on PSA may become masked and inaccessible to the antibodies for detection after binding to the inhibitors. Thus, PSA made up in human serum may have less epitopes available for binding to antibodies; and standards made by this method would result in overestimation of the serum PSA concentration. Another explanation for the kit to kit variation is that some PSA immunoassay methods use a monoclonal/monoclonal antibody sandwich as an immunoassay format, whereas others use a monoclonal/polyclonal antibody sandwich format, with polyclonal antibodies used for detection. Again, if a protease inhibitor would mask some antigenic determinants on PSA, one would expect that a larger amount of polyclonal antibody would bind to free PSA than complexed PSA. This discrepancy from one manufacturer to another makes it impossible to derive a universal cut-off for serum PSA value, where test results reading above the cut-off would indicate abnormality.

Recently, the American Cancer Society recommended that men aged 50 and older be screened for prostate cancer with a PSA test. Therefore, there is a need for a universal reference to standardize PSA immunoassays. A workgroup on the standardization of PSA was formed and recommendations on how to develop a PSA reference standard were made (Rippey JH, Wener MH, Horoszewicz J, Stenman U-H, Klee GG, Bekanger A, Maxim PE, Graves H, Howanitz J, and Nakamura RM. Workgroup #1: Standardization of PSA. *Cancer* 1993, 71:2678). The laboratory of Dr. Stamey at Stanford University has prepared reference material composed of 90% ACT-complexed PSA and 10% free PSA. This material has been sent to various investigators for calibration and results were reported back to Stanford University. Most commercial kits appear to agree reasonably well with this reference standard (Stamey T. Second Stanford Conference on international stanardization of prostate specific antigen (PSA) immunoassays: September 1 and 2, 1994. *Urology* 1995, 45:173).

Free PSA has been purified from both human prostatic tissues and seminal plasma (Wang *et al*, *supra*; and Sensabaugh GF, and Blake ET. Seminal plasma protein p30: simplified purification and evidence for identity with prostate specific antigen. *J Urol* 1990; 144:1523-6; Wang MC, Valenzuela LA, Murthy GP, and Chu TM. A simplified purification procedure for human prostate antigen. *Oncology* 1982; 39:1-5). In light of the finding that the prostate gland also synthesizes ACT and seminal fluid contains both free and complexed forms of PSA (Gershagen S, Schoen S, Cockett ATK, Abrahamsson P-A, and Lilja H. Characterization of the prostatic production of α₁-antichymotrypsin and its implications on the regulation of prostate-specific antigen. *J Urol* 1994 151:366A), a possibility exists that PSA purified from tissues or biological fluids would be contaminated with at least a trace amount of ACT. In fact, Western blot results in our laboratory showed that commercial sources of purified PSA do indeed, contain trace ACT.

As early as 1990, Christensson *et al*, *supra*, showed that PSA formed complexes with ACT, α₂-macroglobulin, and other protease inhibitors. The rate of formation of these complexes, the molar ratio of PSA to inhibitor, and the stability of these complexes are well-established. In the same year, a comparison of two leading commercial PSA immunoassay kits found that, for the same sample, one kit gave values 1.4 to 1.7 times higher than the other (Graves HCB, Wehner N, and Stamey TA. Comparison of a polyclonal and monoclonal immunoassay for PSA: Need for an international antigen standard. *J Urol* 1990, 144:1516-1522). This discrepancy was indicated to be due to a difference in assigned calibrator values for the two kits.

Accordingly, it is evident that from the time of the reports of discrepant results between commercial PSA kits, and the existence of PSA in both free and complexed forms in serum, a source of PSA totally free of ACT and other endogenous protease inhibitors became highly desirable. It is evident that the most desirable source would be through the expression of recombinant material. In this case, the expressed protein can be known at the primary sequence level and prepared in large quantities under controlled conditions. Recombinant PSA expressed in serum-free media would be 100% free-form PSA since such a preparation would be completely free of the protease inhibitors normally associated with biological sources of PSA. In addition, since recombinant PSA would not be human material, there is no chance for contamination by other human viruses (e.g. HIV, CMV, hepatitis B, etc.) that frequently are present in unknown amounts in human samples, particularly of seminal origin. An added feature is the fact that expressed material can be prepared in such a way that it can be easily purified after expression, thus considerably reducing production costs.

Although the successful cloning of cDNA for mature PSA had been reported as early as 1987 (Schulz P, Stucka R, Feldman H, Combriato G, Klobeck H-G, and Fittler F. Sequence of a cDNA clone encompassing the complete mare human Prostate Specific Antigen (PSA) and an unspliced leader sequence. *Nucl Acids Res* 1988, 16:6226); and Lundwall A and Lilja H. Molecular cloning of human prostate specific antigen cDNA. *FEBS Letters* 1987, 214:317-322), extensive literature surveys covering the past eight years have failed to reveal any attempts to express recombinant PSA prior to the present invention. In the original expression cloning of PSA as a fusion protein with *E*. *coli* beta-galactosidase, expressed by the bacteriophage lambda gt11, the mature N-terminus of the protein was included within the fusion molecule, but the complete prepro-signal sequence was not. This region was not identified until much later, and then was only assumed on the basis of genomic cloning and comparative sequence analysis. Subsequent to the present invention, an abstract has published which reports the generation of recombinant PSA in a baculovirus system (Bei R, Kashmiri SVS, Paranavitana C, Milenic D, and Schlom J. A recombinant Prostate Specific Antigen (PSA) produced in a baculovirus expression system *Proceedings of the 86th Annual Meeting of the African Association for Cancer Research* March 18-22, 1995, 36:644).

Thus, to our knowledge, no full-length cDNAs for PSA, bearing the appropriate secretion signal sequences have been expressed prior to the present invention, possibly because of the inconclusive nature of identifying the actual translational start codon and because of the presumed structure of PSA based strictly on comparison with another serine protease, human kallikrein. In addition, although a 7 amino acid propeptide region was described in the literature, there was no conclusive evidence that such a region has functional significance, and the presence of a highly-charged arginine residue preceding the cleavage site that generates the mature N-terminus is highly unusual, since such amino acids are generally aliphatic.

Accordingly, there has been an unsatisfied need for a source of PSA free of protease inhibitors and other contaminants. The absence of reports on the successful recombinant expression of PSA prior to the present invention is therefore evidence of technical problems and the failure of others to accomplish a clearly desired goal.

### SUMMARY OF THE INVENTION

The present invention provides, for the first time, recombinantly expressed prostate-specific antigen (PSA). Such recombinant PSA (rPSA) and immunologically cross-reactive fragments thereof are useful in calibrating ad investigating the results obtained from PSA immunoassays. Further, such rPSA expressed in serum-free media is completely free of protease inhibitors normally associated with biological sources of PSA. Immunological detection of secreted rPSA results in only one major and one minor band, while PSA isolated from human semen consists of a multitude of cross-reactive bands up to approximately 34 kDa.

### BRIEF DESCRIPTION OF THE DRAWINGS

Fig. 1 is a Western blot comparison of rPSA of the present invention and PSA isolated from seminal fluid. The arrow marks the expected location of a 34 kDa protein.

Pig. 2 is a graph plot of a dilution linearity study of two preparations of rPSA. These materials were serially diluted and then assayed using a commercial immunoassay analyzer method.

Fig. 3 is a graph plot of a dose response study of rPSA on a commercial immunoassay analyzer (the Technicon Immuno 1® analyzer). The rPSA calibrators were value-assigned based on commercial calibrators.

Fig. 4 is a graph plot of a dose response study of rPSA using the Hybritech Tandem-E kit.

Fig. 5 is a graph plot of a correlation of PSA assays run on a commercial analyzer using commercial calibrators and calibrators obtained from Stanford University.

Fig. 6 is a graph plot of a correlation of PSA assays run on a commercial analyzer using Stanford calibrators and rPSA calibrators.

Fig. 7 is a graph plot of a correlation of PSA assays run on a commercial analyzer using commercial calibrators and rPSA calibrators.

### DESCRIPTION OF THE PREFERRED EMBODIMENTS

Since 1987, there have been several papers describing the cloning of the human prostate specific antigen gene. However, prior to the press invention, no publications are known detailing the expression of this protein in any prokaryotic or eukaryotic system. The determination of the organization of the prepro-signal sequence preceding the mace PSA form is strictly assumed based on comparison with human kallikrein, another serine protease like PSA. Thus, the requirements for signal processing of the native or recombinant form of PSA is not at all clear. A purpose of this study was to determine if PSA can he expressed and secreted in an expression system and to ask if the existing prepropeptide signal sequence, or the secretion signal minus the propeptide sequence, is sufficient to yield properly-processed PSA. cDNAs were prepared that contained the presumed full-length cDNA with all secretion signals (F1R1 - see below) or contained the full-length cDNA minus the propeptide sequence (F2R1 - see below). These cDNAs were placed into the insect virus expression vector, pVL393. Secreted PSA expressed into media from either F1R1 or F2R1 recombinant cDNAs resulted in the immunological detection of two different-sized expression products, M, 34 kDa. For each of the two different types of clones, a single isolate expressing the complete prepro-signal sequence and mature PSA polypeptide (designated 1R1-1.7A) and an isolate expressing a similar molecule minus the 'pro'-signal sequence (designated 2R1-2.11B) were selected. For 1R1-1.7A, the largest of the 34 kDa bands was considerably more reactive than the smaller, and this latter band may represent incomplete N-linked glycosylation of the potential single site of PSA. Incomplete post-translational modifications are not uncommon in insect systems. For 2R1-2.11B, one or two similar-sized bands were obtained, but these were slightly larger thin those seen for 1R1-1.7A. A possible explanation for this observation includes an inability to process properly molecules that do not contain a *bona fide* signal sequence. This suggests that the 7 amino acid 'pro'-sequence of the PSA signal may indeed be required for proper processing. A further support for these explanations is the several-fold difference in amount of PSA secreted by the insect cells (250 ng PSA per ml of medium for 1R1-1.7A versus 75 ng for 2R1-2.11B). A *Baculovirus* vector that expresses the 1R1-1.7A rPSA antigen has been deposited with the American Type Culture Collection, Rockville, Maryland, USA, and has been given the ATCC designation VR-2496.

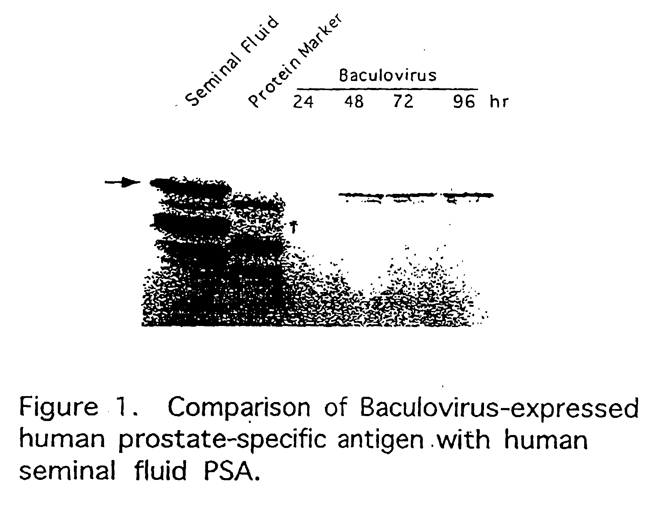

An additional feature of the recombinant system is the observation that immunological detection of secreted rPSA results in only one major and one minor band, while PSA isolated from human semen consists of a multitude of cross-reactive bands up to approximately 34 kDa (see Fig. 1). This indicates the superiority of expressed recombinant forms to obtain homogeneous preparations rather than using native antigen which may result in considerable autolysis.

The rPSA of the present invention has several use. In particular, rPSA is used as a calibrator for PSA immunoassays. Such immunoassays will usually follow a sandwich format using two antibodies, one for detection and one for separation. Typically, both antibodies are monoclonal antibodies, or one antibody is monoclonal and the other is polyclonal. As more is understood concerning the clinical significance of measuring free and/or protease inhibitor-complexed PSA and/or the ratio of complexed to free PSA, rPSA will be useful in evaluating and formulating calibrator compositions. Additionally, rPSA can be used to immunize appropriate host animals in the generation of polyclonal and monoclonal antibodies, and for other purposes evident to those skilled in the art.

It will be understood than throughout this disclosure, the term recombinant PSA (or rPSA) is intended to include immunologically cross-reactive fragments of the recombinantly expressed polypeptide. Such fragments can be prepared in any known manner, such as by proteolytic, autolytic, or chemical digestion or cleavage; recombinant expression of partial cDNA; and the like Immunological cross-reactivity will be understood to mean the ability of a fragment to be hound to an analytically significant degree by relevant anti-PSA antibodies. More usually, an immunologically cross-reactive fragment will exhibit cross-reactivity relative to full length rPSA of about 50% or greater, more normally more than about 30%, and will be at least about 10%. As is understood in the art, such fragments will normally be at least about ten amino acids in length, and can be as small as about four amino acids.

The present invention will now be illustrated, but is not intended to he limited by, the following examples.

### EXAMPLES

### MATERIALS AND METHODS

### Recombinant Expression of PSA

Five nanograms (5 ng) of first-strand normal prostate cDNA (Clontech Laboratories, Palo Alto, California, USA) were incubated with one of two forward primers
F1 = SEQ ID NO:1
F2 = SEQ ID NO:2
and a reverse primer
R1 = SEQ ID NO:3. 
SEQ ID NO:1 5'-GGATCCTGTGTCACCATGTGGGTCCCGGTTGTCTTCCTCACCC
TG

SEQ ID NO:2 5'-GGATCCTGTGTCACCATGTGGGTCCCGGTTGTCTTCCTCACC
CTGTCCGTGACGTGGATTGGTGCTATTGTGGGAGGCTGGGAGAAGCATTCC

SEQ ID NO:3 5'-GCGGCCGCATCATTAATGGTGATGGTGATGATGGGGGTTGGC
CACGATGGTGTCCTTGATCCACTT
 The reverse primer R1 include six terminal codons that code for histidines that could permit PSA binding to a matrix containing either imidodiacetic acid-Zn²⁺ or nitrilotriacetic acid-Ni²⁺ chelates and its gentle removal by increasing concentrations of competitive ligand, such as imidazole or histidine) oligonucleotide primers in a 400 µL reaction containing 10 mM Tris-HCl, pH 8.3, 50 mM KCl, 1.5 mM MgCl₂, 0.01% Triton-X-100, 0.1% bovine serum albumin, 200 µM deoxyribonucleotide triphosphates and 12.5 units of AmpliTaq DNA polymerase (Perkin Elmer Cetus, Norwalk, Connecticut, USA). Amplification was for a total of 30 cycles: the first cycle was for 3 min at 96°C, 45 sec at 55°C, 30 sec at 72°C; each of the 28 subsequent cycles were for 45 sec at 96°C, 30 sec at 55°C, 45 sec at 72°C and 1 cycle was for 45 sec at 96°C, 30 sec at 55°C, 2 min at 72°C. The reaction was then extracted with phenol/chloroform and amplified DNA was precipitated by addition of 2.5 vol of 95% ethanol. DNA pelleted from ethanol was resuspended in distilled water, digested with BamHI and Not I restriction endonucleases (underlined in the oligonucleotide primer sequences above), and electrophoresed on a 1% low-melting agarose gel (1:1 mixture of LGT and NuSieve GTG agaroses). The 800 bp DNA segment corresponding to the expected size of PSA was purified by phenol extraction, precipitated and resuspended in distilled water before ligating to phosphatased and BamHI-Not I-cleaved pVL1393 baculovirus transfer vector (Invitrogen, San Diego, California, USA). Ligation products were transformed into chemically-competent *E*. *coli* DH5α cells for 1 min at 42°C, then plated onto LB-agar plates containing 50 µg/mL of sodium ampicillin. Transformants were screened from 1.5 mL overnight cultures by the alkaline denaturation method. DNAs containing the predicted 0.8 kb insert were sequenced at the insert end by priming within the baculovirus vector. Samples with correct sequence were co-transfected with linearized wild-type baculovirus DNA into *Spodoptera frugiperda* Sf9 cells, then plated in soft agarose for detection of inclusion-body-minus recombinants. Five independent recombinants were selected for growth in small-scale culture (T-25 flasks) and assayed for secreted PSA.

Filtered supernatants from small-scale cultures were mixed with a 0.2 volume of 5X Laemmli denaturing buffer containing 5% beta-mercaptoethanol and 5% sodium dodecyl sulfate, then boiled to denature and reduce proteins. Samples were electrophoresed on denaturing 8-16% gradient polyacrylamide gels, and polypeptides were transferred to PVDF membranes for immunological detection. Antibodies used were polyclonal goat anti-human PSA, followed by polyclonal swine anti-goat IgG labelled with alkaline phosphatase. Colorimetric detection was with NBT and BCIP.

### Technicon Immuno 1® PSA Assay

The Technicon Immuno 1 PSA assay was conducted according to instructions provided by the manufacturer (Bayer Corporation, Business Group Diagnostics, Tarrytown, New York, USA). The assay employs a monoclonal anti-PSA antibody conjugated to fluorescein (R1), an affinity-purified polyclonal antibody conjugated to alkaline phosphatase (R2) and magnetic particles as a solid phase for separation. Briefly, 20 µL of a test specimen was incubated with R1 and R2 at 37°C in a reaction tray cuvette on the Technicon Immuno 1 analyzer. During this reaction, the antibodies in R1 and R2 bind to different epitopes of the PSA molecule and form an immune complex. This complex then binds to the magnetic particles which had been coated with antibodies to fluorescein via a fluorescein-antifluorescein linkage. The magnetic particles are held in the cuvettes on the instrument and washed to remove the unbound components. After washing, para-nitrophenol phosphate (PNPP) is added to the magnetic particles. The enzyme associated with the particles hydrolyzes the substrate to produce a color which is measured at 405 or 450 nm. The rate of color formation is directly proportional to the concentration of PSA in the test sample. The PSA concentration in a specimen is derived from comparing the rate to those stored in a standard curve. The entire procedure is carried out by the automated analyzer and takes 38.5 minutes.

### Hybritech Tandem-E PSA Assay

The Hybritech Tandern-E assay is a non-isotopic enzymatic solid-phase sandwich assay using two monoclonal antibodies for capture and detection of PSA. One anti-PSA antibody is coated onto plastic beads while the other is conjugated to alkaline phosphatase. Both antibodies recognize distinct epitopes of PSA and are non-competing. According to the manufacturer's instructions, 100 µL of test specimen is mixed in a polystyrene round bottom 12x75 mm test tube with 100 µL of anti-PSA antibody conjugated to alkaline phosphatase. PSA in the specimen binds to the antibody conjugate and is captured by a bead coated with another anti-PSA antibody. After 2 hours of incubation at room temperature, the bead is washed with 6 mL total volume of wash solution provided by the manufacturer. After the washing protocol, 200 µL of PNPP substrate was added to the bead and incubated for 30 minutes PNPP substrate (Pierce, Rockford, Illinois, USA), 1.66 g/L in diethanolamine buffer, was used in place of substrate from Hybritech. Color development of the reaction was terminated by addition of 1.5 mL of 3 N NaOH. An aliquot of 200 µL was transferred to a microtiter plate and absorbance at 405 nm was measured using a plate reader (Molecular Devices, Sunnyvale, California, USA). Concentrations were calculated from either a point-to-point or cubic-fit of the standard curve using curve fitting programs.

### Analytical Recovery with Recombinant PSA

Recombinant PSA was diluted in either phosphate-buffered saline (PBS); 6% BSA, 111 mM NaCl, 25 mM Tris, 0.1% NaN₃, 0.2% Proclin 150, pH 7.4 (BSA Buffer); or a pooled normal human serum. The diluted materials were assayed on either the Technicon Immuno 1 assay method or Hybritech Tandem-E method. The original concentrations of recombinant PSA prior to dilution are shown in Table 1A and 1B.

### Dilution Linearity

Two preparations of recombinant PSA with concentrations of 75 and 239 µg/L were used to test for dilution linearity studies. The antigens were serially diluted to 2, 4, 8, 16, 32, and 64 fold with BSA Buffer. Samples were assayed using the Technicon Immuno 1 PSA method.

### Western Blots

Pre-casted gels containing a gradient of 4% to 20% acrylamide were purchased from Integrated Separation Systems, Natick, Massachusetts, USA. Suffers for gel electrophoresis (Tris-glycine buffer) and Western blotting (Transfer buffer) were made from pre-mixed packages from Pierce. Nitrocellulose membranes were from Hoefer Scientific Instruments, San Francisco, California, USA. BLOTTO blocking buffer was obtained from Pierce. Monoclonal and polyclonal antibodies to PSA were obtained from Bayer Corporation, Business Group Diagnostics, Elkhart, Indiana, USA. BCIP/NBT tablets, alkaline phosphatase-conjugated goat anti-mouse IgG and alkaline phosphatase-conjugated rabbit anti-goat IgG were purchased from Sigma Chemical Co., St. Louis, Missouri, USA)
The gels were soaked in Transfer buffer (25 mM Tris, 192 mM glycine, 20% methanol, pH 8.0) for 10 minutes before transferring proteins to nitrocellulose membranes at a constant current of 200 milliamps for 2 hours. After blocking with BLOTTO for 30 minutes, the membranes were incubated overnight with 10 mL of either monoclonal PSA or polyclonal PSA at a concentration of 1 mg/L. The membranes were washed 3 times with PBS containing 0.02% sodium azide followed by incubation with 10 mL of either goat anti-mouse polyvalent (IgG, IgA, IgM) alkaline phosphatase or rabbit anti-goat IgG (whole molecule) alkaline phosphatase at a 1:1000 dilution for 1 hour. After the first antibody incubation, the membranes Were washed 3 times with PBS containing 0.02% sodium azide followed by 1 wash with distilled water. One BCIP/NBT tablet was dissolved in 10 mL of distilled water for each membrane. The membranes were incubated in the substrate for 10 minutes.

Western blots comparing semical fluid PSA with the 1R1-1.7A rPSA are shown in Fig. 1 of the drawings. rPSA appears as one major and one minor band, whereas seminal fluid PSA consists of a multitude of bands.

### Comparison of Calibrators

Fifty (50) serum specimens from patients with prostate cancer were tested with the Technicon Immuno 1 PSA assay. The instrument was calibrated with three sets of standards: Technicon Immuno 1 PSA Set Point® Calibrators, rPSA, and a set of calibrators made up of 90% ACT-complexed/10% free PSA (from Dr. T. Stamey, Stanford University, Palo Alto, California, USA). Calibration curves were curve-fitted using a cubic-fit through zero algorithm.

### N-Terminal Amino Acid Sequencing

In general, the affinity purified rPSA and commercial native PSA were separated by SDS-PAGE under reducing conditions and electroblotted on PVDF membrane. The bands were cut out for N-terminal amino acid sequencing. Also, the band was confirmed by gold staining method and Western blotting analyses. Briefly, purified rPSA and native PSA (Scripps Laboratories, Inc., San Diego, California, USA) were treated with an equal volume of 2X Tris-glycine SDS-PAGE sample buffer containing 125 mM Tris, 2% SDS, 20% glycine, pH 6.8, with 50 mM DTT, and boiled for 3 min. 25 µg of rPSA and 38 µg of native PSA were electrophoresed into each precasted 4-20 % SDS-PAGE 2-D well gel (Integrated Separation Systems, Natick, Massachusetts, USA) with the electrophoresis buffer (25 mM Tris, 196 mM glycine, 0.1% SDS, pH 8.3, Pierce, Rockford, Illinois, USA). The gels were washed in PBS (5 prime → 3 Prime, Inc., Boulder, Colorado, USA) for 10 min at room temperature with gentle rocking. The separated polypeptides in the washed gels were electrotransferred onto PVDF membranes (Sigma, St. Louis, Missouri, USA) with the 1X transfer buffers (Anode buffer #1, Anode buffer #2 and Cathode buffer, Pierce) at 125 mA for 45 min at room temperature using semi-dry apparatus (Integrated Separation Systems-Enprotech, Natick, Massachusetts, USA). The blots were then washed in water for 3 times for 5 min each, stained with 0.1% Coomassie Blue R-250 (BioRad, Melville, New York, USA) in 50% methanol, destained with 50 % methanol, and finally rinsed with distilled water. The bands were cut off from PVDF membranes individually for amino acid N-terminal sequencing.

All of the PSA bands on the PVDF membrane were confirmed by Western blotting analysis as described by Towbin et al (Towbin, H., Staehelin, T. and Gordon, J., 1979, Electrophoretic transfer of proteins from polyacrylamide gels to nitrocellulose sheets: procedures and some applications. *Proc*. *Natl*. *Acad*. *Sci*. *USA* 76:4350-4354). The electroblotted PVDF membranes were blocked in PBS/3% BSA (Sigma) at room temperature for 1 hour. Primary antibody, goat anti-PSA polyclonal antibody was obtained from Bayer Corporation. Secondary antibody is an alkaline phosphatase-conjugated rabbit anti-goat IgG polyclonal antibody (Sigma). The blots were incubated with primary and secondary polyclonal antibodies (1:1000 dilution with 3% BSA) at 37°C for 2 hours, respectively, and washed with PBS containing 0.05% Tween 20 (Integrated Separation Systems) for 10 min at room temperature after incubation of PSA polypeptides with the primary and secondary antibodies. BCIP/NBT substrate (Sigma) were used for color development of the blots.

The PSA polypeptide bands were confirmed by Western blotting and used for amino acid N-terminal sequencing performed on the Applied Biosystems 477A Protein Sequencer. Edman chemistry system was used. The coupling and cleavage steps were performed in the Gas phase. The PTH amino acids were determined by an on-line HPLC, the Applied Biosystems 120A PTH analyzer. Data were integrated by a Nelson Analytical Chromatography Data System. Native PSA (Scripps Laboratories, Inc.) was used as a control. Tile N-terminal amino acid sequences of each PSA band were compared with the derived amino acid sequences of cloned PSA cDNA.

### RESULTS

### Analytical Recovery

Recombinant PSA was diluted 2-fold in either PBS, BSA Buffer, or a pooled normal human serum. The diluted recombinant antigen was measured using either the Technicon Immuno 1 assay method or the Hybritech Tandem-E assay method. There was no significant differences in the recovery of recombinant PSA when diluted in either PBS or BSA Buffer (Table 1A). In addition, recombinant PSA is reactive with both Technicon Immuno 1 and Hybritech Tandem-E methods (Table 1B). However, the recovery of the antigen in serum was 49.8% using the Technicon Immuno 1 assay method and 54.4% using the Hybritech Tandem-E assay method.

The decrease in recovery is not unexpected and the same phenomenon has been reported by Graves *et al* and Zhou *et al* (*supra*). One explanation for this poor recovery is that when PSA complexes with protease inhibitors, such as α₂-macroglobulin, the PSA epitopes are masked and become inaccessible to the antibodies and therefore cannot be detected by immunoassays. These data show that rPSA and PSA purified from seminal fluid are similar in reactivity with protease inhibitors and the recovery of rPSA in sera is similar for both the Technicon Immuno 1 PSA Assay and the Hybritech Tandem-E PSA Assay.

**Table 1A**

| Comparison of Recovery of Recombinant PSA in PBS and BSA | |
|---|---|
| 1R1-1.7A | Immuno-1 (mg/L) |
| in PBS | 2.61 |
| in BSA | 2.67 |
| Recovery | 102 % |

**Table 1B**

| Comparison of Recovery of Recombinant PSA in BSA and Sera by Technicon Immuno 1 Method and Hybritech Tandem-E Method | | |
|---|---|---|
| 1R1-1.7A | Immuno 1 (µg/L) | Hybritech (µg/L) |
| in BSA | 229.79 | 164.89 |
| in Sera | 114.50 | 89.63 |
| Recovery | 49.8% | 54.4% |

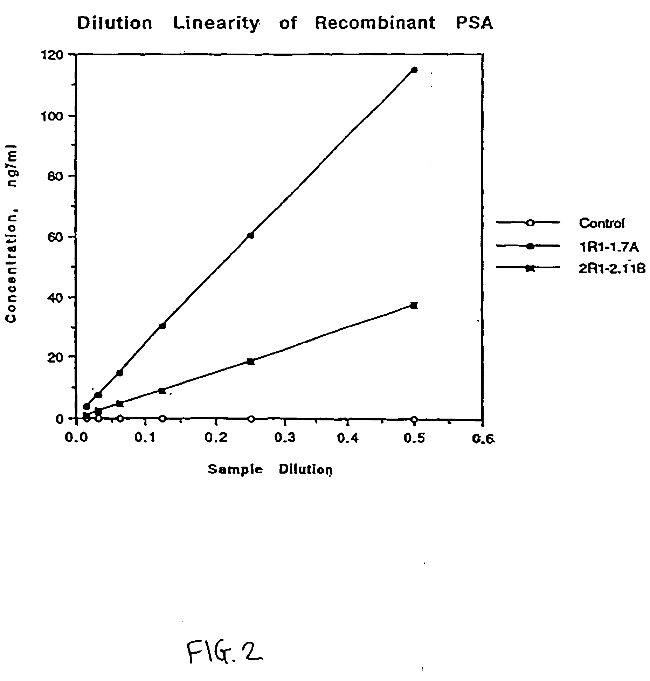

The two recombinant PSA preparations were serially diluted with BSA Buffer as described in Materials and Methods. The diluted samples were assayed on Technicon Immuno 1 PSA method. Figure 2 shows that both recombinant PSA preparations, 1R1-1.7A and 2R1-2.11B, diluted linearly.

### Dose Response with Commercial Kits

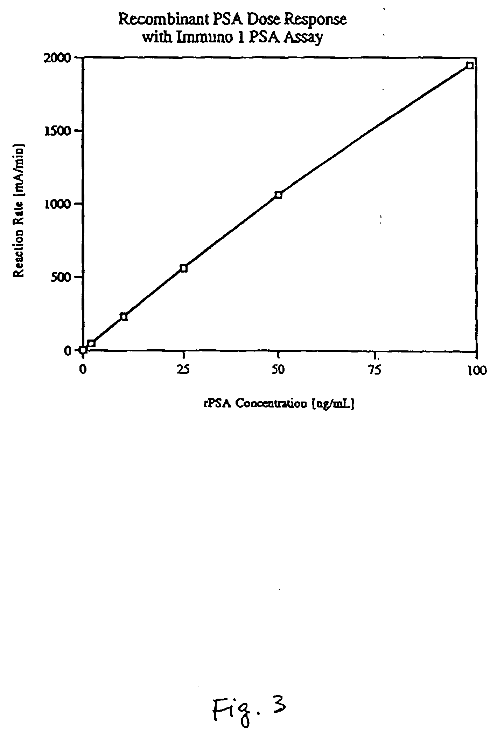

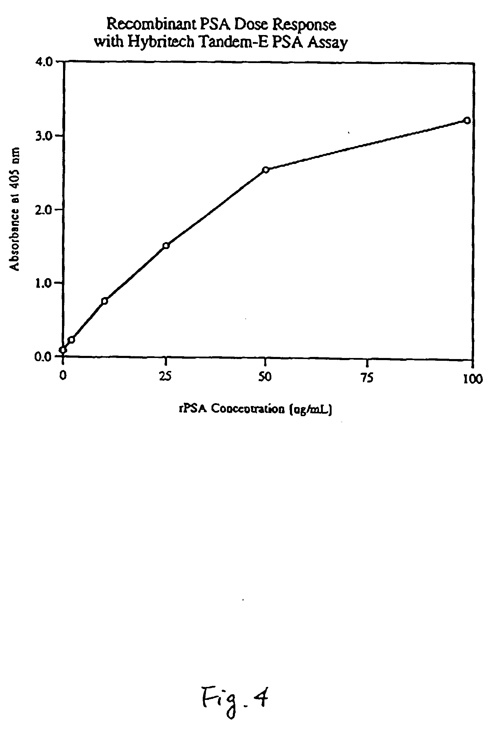

Figures 3 and 4 show dose response curves produced by assaying rPSA with the commercial Technicon Immuno 1 assay kit and the Hybritech Tandem-E assay kit.

### Correlation of Calibrators

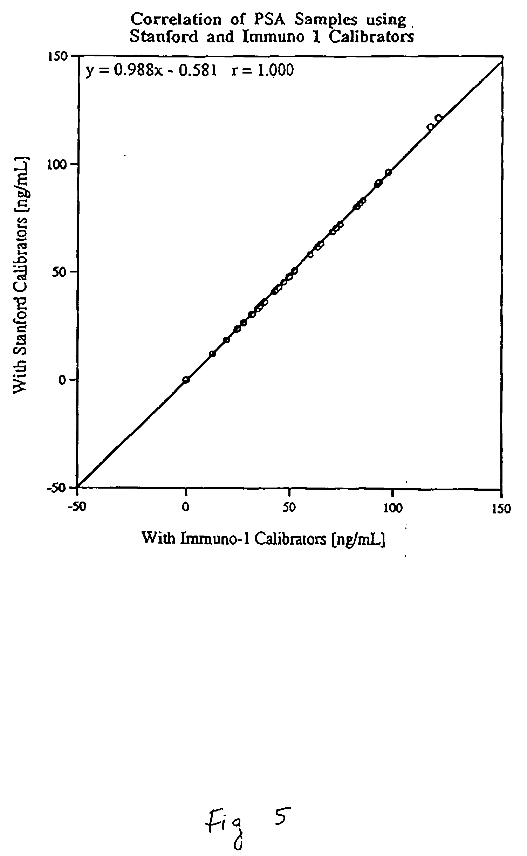

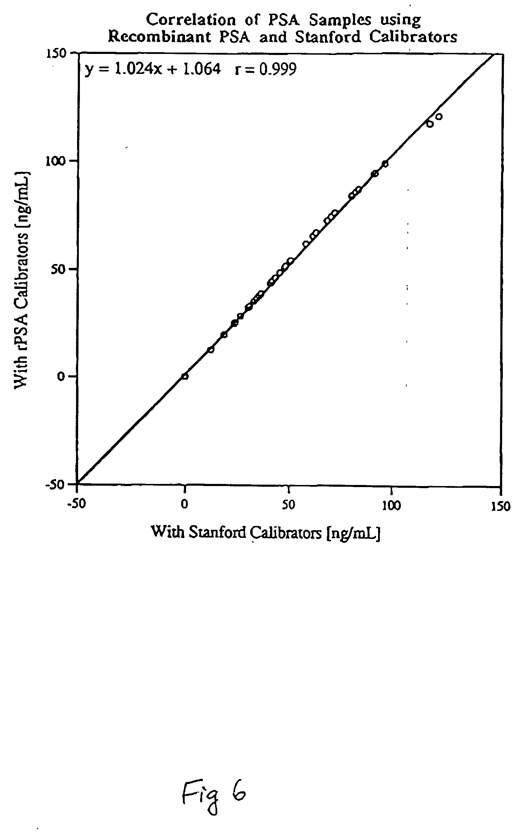

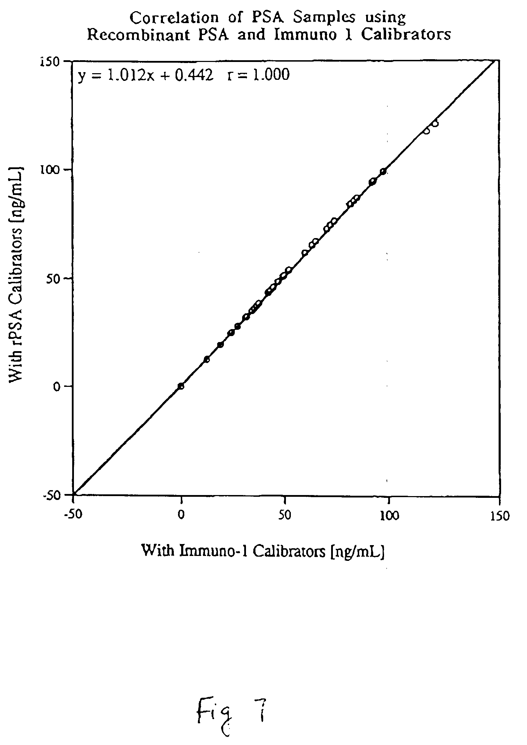

Results are shown in Figures 5,6 and 7. Correlation of the samples using the 3 calibrators yields the following regression equations:
y = 1.012 x + 0.442 r = 1.000 recombinant PSA vs. Immuno 1 PSA
y = 0.988 x + 0.581 r = 1.000 Stanford PSA vs. Immuno 1 PSA
y = 1.024 x + 1.064 r = 0.999 recombinant PSA vs. Stanford PSA
These results show that PSA values in serum samples derived from these different standards agree well with one another. The Immuno 1 Set Point calibrators were male from PSA purified from human seminal fluid. These data suggest that the recombinant PSA would have comparable antigenic properties as the purified human material and would be suitable for use as calibrators for PSA assays. However, it is unexpected that specimen PSA values derived from the Stanford calibrators which are made up of 90% ACT-complex and 10% free PSA would also agree with those from rPSA. This suggests that the Technicon Immuno 1 PSA assay method measures complexed and free PSA in equal molar quantities.

### Confirmation of Amino Acid Sequence

The affinity purified rPSA was separated by SDS-PAGE under non-reducing conditions into four bands with the following molecular weights: 32.9, 30.0, 25.3 and 18.4 kDa. N-terminal amino acid sequences of recombinant PSA showed that the primary sequences of higher molecular weight bands 1 and 2 matched human native PSA precursor beginning at residue 24, arginine. This arginine residue is part of the propeptide (activation polypeptide from residues 18 to 24) based on the sequence of the full length PSA cDNA (Lundwall and Lilja, 1987. Molecular cloning of human prostate specific antigen cDNA. *FEBS Letters*, 214(2):317-322), and on the mature N-terminus of the PSA polypeptide that starts with the aminoacid isoleucine (Watt et al., 1986. Human prostate-specific antigen: Structural and functional similarity with serine proteases. *PNAS* 83:3166-3170). These two bands possess the same N-terminus, but their molecular sizes are slightly different on the SDS-PAGE and Western blots. This may be due to differences in polypeptide glycosylation (Bei, R., Kashmiri, S.V.S., Paranavitana, C., Milenic, D. and Schlom, J., 1995, *Proc*. *The Amer*. *Assoc*. *for Cancer Res*., 36:644, Abstract #3832). Bands 3 and 4 of rPSA are internal sequences and their N-terminal begin at residues 82 (histidine) and 108 (asparagine), respectively. The calculated molecular weights for these two bands based on amino acid sequences are 19.9 kDa and 16.8 kDa, respectively.

The present invention has been particularly described and exemplified above. Clearly, many other variations and modifications of the invention can be made without departing from the spirit and scope hereof.

## Claims

1. Recombinant PSA and immunologically reactive fragments thereof.

2. Recombinant PSA of claim 1 wherein said fragments comprise at least four amino acids.

3. Recombinant PSA of claim 1 expressed by a vector having a cDNA insert selected by PCR using either SEQ ID NO:1 and SEQ ID NO:3 or SEQ ID NO:2 and SEQ ID NO:3 as the primer pair.

4. Recombinant PSA of claim 1 expressed by the vector ATCC VR-2496, and immunologically cross-reactive fragments thereof.

5. Recombinant PSA of claim 4 wherein said fragments comprise at least four amino acids.

6. In an immunoassay method for the quantitative determination of PSA in a biological test sample wherein the test sample is combined with immunoassay reagents comprising a labeled conjugate and wherein the response of the labeled conjugate is compared to a standard curve established by performing the assay on one or more calibrators,
the improvement which comprises employing recombinant PSA or an immunologically cross-reactive fragment thereof in said one or more calibrators.

7. The immunoassay method of claim 6 wherein said fragments of rPSA comprise at least four amino acids.

8. The immunoassay method of claim 7 wherein said rPSA is expressed by a vector having a cDNA insert selected by PCR using either SEQ ID NO:1 and SEQ ID No:3 or SEQ ID NO:2 and SEQ ID NO:3 as the primer pair.

9. The immunoassay method of claim 6 wherein said rPSA is expressed by the vector ATCC VR-2496.

10. The immunoassay method of claim 9 wherein said fragments comprise at least four amino acids.
